# EUROPEAN PATENT APPLICATION

(11) **EP 4 589 003 A1**
(43) Date of publication of application: **23.07.2025**
(21) Application number: 24208760.9
(22) Date of filing: 24.10.2024
(51) Int. Cl.: C12N 5/071

(54) **RESPIRATORY ORGANOIDS WITH EXTERNALLY ATTACHED CILIATED EPITHELIAL CELLS AND METHOD FOR PREPARING THE SAME**

(30) Priority: 19.01.2024 KR 20240008778
(71) Applicant: Gil Medical Center, Incheon 21565 (KR)
(72) Inventor: SON, Kuk Hui, 21565 INCHEON (KR); LEE, Jin Woo, 21565 INCHEON (KR); CHOI, Seon Young, 21565 INCHEON (KR); KIM, Kun Woo, 21565 INCHEON (KR)
(74) Representative: Regimbeau

(57) **Abstract**

The present disclosure relates to a method for preparing a respiratory organoid by proliferating and differentiating respiratory epithelial cells. Unlike the conventional respiratory organoids in which epithelial cells existing in the apical region of the respiratory organ are generated on the interior of the organoid, the respiratory organoid of the present disclosure is shown in a form in which ciliated structures, which are epithelial cells, are located in the outer layer of the organoid, secretory cells are present on the interior, and basal stem cells existing at the base of respiratory tissue are also present on the interior of the organoid. It has a form similar to an actual respiratory tissue, and it was observed that the epithelial cells exhibited normal function, such as motility of ciliated cells. Therefore, when an organoid is prepared using the preparation method of the present disclosure, a respiratory organoid that can well mimic actual tissue can be prepared.

## Description

### [Technical Field]

The present disclosure relates to respiratory organoids and a method for preparing the same. The present disclosure specifically relates to respiratory organoids with externally attached ciliated epithelial cells and a method for preparing the same.

### [Background Art]

Organoids, also called as biomimetic or organ-like bodies (mini-organs), refer to small cultures that reproduce both the form and function of a tissue or organ by three-dimensionally culturing, aggregating, or recombining cells isolated from stem cells or organ-derived cells. These organoids contain several specific cell populations that make up an organ or tissue, have a form and structural organization similar to an actual tissue or organ, and can reproduce the special functions of each organ. Organoids are formed through a series of common processes. Cells with the same function are grouped together and placed in an appropriate location, and after the cell compartments are separated, more detailed differentiation occurs. Organoids are similar to actual organs and can facilitate research *in vitro* that is difficult to implement in animal models, such as regulating molecular signals, making them very useful for basic research, as well as human developmental processes, establishing disease models, and drug effectiveness. Organoids are very useful for basic research because they can be used to conduct studies that are difficult to implement in animal models *in vitro,* such as molecular signal regulation, in a form similar to actual organs, and are a technology that can be very useful in various fields, such as human developmental processes, disease model establishment, screening for drug efficacy, cell therapy development, *etc.*

A support or a scaffold which plays a supporting role so that cells can adhere and grow in the process of preparing organoids not only plays a very important role in biological tissue engineering, but also plays a significant role in the growth of cells disseminated in a porous structure and cells migrating from the surrounding tissue. Most of the cells in the human body are adherent cells that grow while being adhered, and if there is no place for them to adhere, the cells cannot grow and eventually die. Therefore, the support should provide a suitable environment for cell adhesion, differentiation, growth and cell migration. Such a support can be made of various materials, and research to develop a support using a natural material, a synthetic polymer, bioceramics, and a polymer-ceramic composite material is being actively conducted. Accordingly, organoids that mimic various organs have been developed so far, and these are mainly cultured in Matrigel, a three-dimensional culture environment.

However, although Matrigel is widely used for organoid culture, it is derived from mouse sarcoma, and there is no particular substitute. Therefore, despite the high cost, there is no choice but to rely on Matrigel. In addition, certain components dominate in Matrigel and there is a limitation in reflecting the tissue-specific characteristics. In order to complement and replace this limitation, decellularization of tissues and organs has been studied as a promising method for preparing functional supports and scaffolds for cell culture and transplantation, and there is an emerging need for such a method. However, the organoids prepared by the current organoid culture technology have many differences from the actual human tissues in terms of the degree of differentiation and functions, and thus the development of a technology for culturing more mature organoids is required.

Studies for establishing various organoids have been conducted in addition to the above, and lung organoids have also been developed. As prior art related to the method for preparing a lung organoid, there are the following documents that disclose a method for preparing a lung organoid from human pluripotent stem cell (hPSC): Development, 144: 986-997, 2017 and Nature cell biology, 19(5): 542-549, 2017.

However, respiratory organoids prepared by the conventional preparation methods have epithelial cells located on the interior of the organoids, and thus, when evaluating tissue damage caused by toxic substances or harmful bacteria introduced through respiration using these organoids, an additional process of injecting the harmful substances into the organoids is required, which is a disadvantage. In order to solve this problem, although apical-out respiratory organoids with externally located ciliated cells have been prepared, there is a limitation in that goblet cells were not present in these organoids, making it difficult to accurately identify infection responses of cells caused by pathogen exposure (Scientific Reports, 12:7673, 2022).

Accordingly, the present inventors have made an attempt to control the polarity of basal stem cells derived from the respiratory organ by controlling the components of the extracellular matrix, and have confirmed that apical-out respiratory organoids, in which ciliated epithelial cells are located on the exterior, basal stem cells are located on the interior, and goblet cells, which are secretory cells, are present within the organoids, can be prepared by three-dimensionally culturing and differentiating organoids using laminin instead of Matrigel, i.e., respiratory organoids that better embody the structure of an actual respiratory tissue, thereby completing the present disclosure.

### [Disclosure]

### [Technical Problem]

It is an object of the present disclosure to provide a method for preparing respiratory organoids with externally attached ciliated epithelial cells and respiratory organoids obtained by the preparation method.

### [Technical Solution]

In order to achieve the object above,
one aspect of the present disclosure provides a method for preparing a respiratory organoid, in which ciliated epithelial cells are present on the exterior, including:
(a) culturing respiratory cells on a plate coated with an extracellular matrix material;
(b) obtaining the cultured cells of Step (a) and removing the expansion medium by centrifugation, thereby obtaining a cell pellet; and
(c) resuspending the cell pellet in a differentiation medium, and then inoculating and culturing a certain number of cells on a plate to be differentiated.

Additionally, another aspect of the present disclosure provides a respiratory organoid prepared by the above method.

Additionally, still another aspect of the present disclosure provides a method for evaluating the toxicity of a harmful substance, including:
(a) treating a respiratory organoid prepared by the above method with a harmful substance;
(b) measuring the viability of the respiratory organoids treated with the harmful substance in Step (a) and the respiratory organoids not treated with the harmful substance; and
(c) determining the toxicity of the harmful substance by comparing the viability of cells measured in Step (b).

### [Advantageous Effects]

In the present disclosure, respiratory organoids were prepared by proliferating and differentiating normal human bronchial epithelial cells (NHBE cells), and the respiratory organoids prepared by the method of the present disclosure have an apical-out form, in which ciliary structures are present on the exterior of the organoids, goblet cells, which are secretory cells, are located on the interior of the organoids, and basal stem cells are present on the interior. These organoids have a form more similar to that of the organs in the body, and considering the purpose of organoids to mimic actual organs, it can be expected that reactions as close as possible to those occurring in an actual organ can be simulated/implemented.

### [Brief Description of Drawings]

FIG. 1 shows the results of observing organoids according to the differentiation period after seeding 500 or 1,000 cells cultured in an expansion medium into a 96-well plate.
FIG. 2a shows the results of observing respiratory organoids on day 21 of differentiation. FIG. 2b is an enlarged view of FIG. 2a.
FIG. 3 shows the results of performing a Live/dead staining assay using respiratory organoids during the differentiation process.
FIG. 4 shows the result of confirming the cell composition of organoids through immunofluorescence staining after preparing respiratory organoids with the medium composition according to Table 1, and ciliated epithelial cells, secretory cells (goblet cells), and cell nuclei can be confirmed.
FIG. 5 shows the result of confirming the distribution of basal stem cells in the organoids through immunofluorescence staining after preparing respiratory organoids with the medium composition according to Table 1.
FIG. 6 shows the result of confirming the cell composition of organoids through immunofluorescence staining after preparing respiratory organoids with the medium composition according to Table 2, and ciliated epithelial cells, secretory cells (goblet cells), and cell nuclei can be confirmed.
FIG. 7 shows the result of confirming the distribution of basal stem cells in the organoids through immunofluorescence staining after preparing respiratory organoids with the medium composition according to Table 2.

### [Detailed Description of Preferred Embodiments]

Hereinafter, the present disclosure will be described in detail.

The present disclosure provides a method for preparing a respiratory organoid, in which ciliated epithelial cells are present on the exterior, including:
(a) culturing respiratory cells using an expansion medium on a plate coated with an extracellular matrix material;
(b) obtaining the cultured cells of Step (a) and removing the expansion medium by centrifugation, thereby obtaining a cell pellet; and
(c) resuspending the cell pellet in a differentiation medium, and then inoculating and culturing a certain number of cells on a plate to be differentiated.

The organoid, which is a mimic organ created by culturing or recombining cells isolated from stem cells or organ-derived cells, refers to an artificial, *in vitro* construct created to mimic or resemble the functionality and/or histological structure of an organ or portion thereof.

The respiratory organ of the respiratory organoid may be a part of the upper and lower respiratory tracts including the lungs, and may preferably be a lung.

The respiratory cell may be any stem cell that can differentiate into a respiratory epithelial cell having respiratory cilia, and may be a normal human bronchial basal epithelial cell (NHBE cell).

The extracellular matrix material of Step (a) above may be laminin, collagen I, or collagen IV. The polarity of stem cells varies depending on the extracellular matrix material exposed to the cells. Although conventional methods often use Matrigel, Matrigel is expensive, thus, it costs a tremendous amount to produce organoids and also takes a long time, accordingly, it was attempted to exclude the use of Matrigel in the present disclosure.

The expansion medium used in Step (a) above may include A8301, Y-27632, CHIR99021, and SB202190.

As used herein, the term "culture medium" means a material that enables the maintenance of cell growth and survival, and includes all conventional culture media used in the art including components suitable for culturing cells. The culture medium and culture conditions may be selected depending on the type of cultured cells. As examples of basal medium for culturing cells, Dulbecco's modified eagle's medium (DMEM), minimal essential medium (MEM), basal medium eagle (BME), RPMI1640, F-10, F-12, Glasgow's minimal essential medium (GMEM), Iscove's modified Dulbecco's medium, bronchial epithelial growth medium (BEGM), *etc.* may be used, and antibiotics such as penicillin-streptomycin or supplements, etc. may be further added as needed. In the present disclosure, it may include A8301, Y-27632, CHIR99021, and SB202190 in bronchial epithelial growth medium (BEGM).

A8301 (A83-01) is known to be used as an essential compound for stem cell expansion as an inhibitor of the TGFβ/SMAD signaling pathway. In the present disclosure, A8301 may be added at a concentration of 0.5 to 2 uM, preferably at a concentration of 0.75 to 1.5 uM, and more preferably at a concentration of 1 to 1.3 uM. An excessive inhibition of the TGFβ/SMAD signaling pathway may lead to hyperplasia of stem cells.

It is known that Y-27632 plays a role in maintaining the stem cell capacity of primary cells as an inhibitor of the ROCK signaling pathway. In the present disclosure, Y-27632 may be added at a concentration of 2 to 10 uM, preferably at a concentration of 3 to 7 uM, and more preferably at a concentration of 4 to 6 uM. The ROCK signaling pathway plays a role in the regulation of stem cell differentiation, thus an excessive or insufficient inhibition may prevent normal differentiation of stem cells.

CHIR99021 is a compound that activates the Wnt signaling pathway and inhibits the GSK3β signaling pathway. In the present disclosure, CHIR99021 may be added at a concentration of 0.5 to 3 uM, preferably at a concentration of 0.5 to 2 uM, and more preferably at a concentration of 0.5 to 1 uM. Since the Wnt signaling pathway plays a role in the regulation of cell differentiation, if the concentration of CHIR99021 is low or absent, basal stem cells may not be maintained.

SB202190, which is a compound known as an inhibitor of the MAPK signaling pathway, is used when proliferating stem cells. In the present disclosure, SB202190 can be added at a concentration of 0.1 to 2 uM, preferably at a concentration of 0.3 to 1.5 uM, and more preferably at a concentration of 0.3 to 1 uM. An excessive inhibition of the MAPK signaling pathway may inhibit mucus production, which may prevent normal differentiation into secretory cells (goblet cells).

The differentiation medium used in Step (c) above may be used without limitation as long as it is known as a differentiation medium for respiratory epithelial cell proliferation, and may preferably be Air-Liquid Interface Medium (Promocell, #C-21080) or PneumaCult-ALI medium (Stemcell Technologies, #05001).

The differentiation medium may include hydrocortisone and heparin in PneumaCult-ALI medium.

Hydrocortisone is an analog of cortisol, a steroid hormone, and plays a role in promoting differentiation. In the present disclosure, hydrocortisone may be contained in the medium at a concentration of 0.5 to 1.0 µg/ml, and preferably 0.75 to 1.0 µg/ml. Hydrocortisone plays a role in promoting the growth and differentiation of respiratory cells, but if used in excess, gene expression and physiological characteristics of cells may alter, which may prevent normal differentiation.

Heparin is a polysaccharide with anticoagulant action and is currently widely used as an anticoagulant, and plays a role in maintaining the stability of growth factors, such as fibroblast growth factor (FGF) and epidermal growth factor (EGF). In the present disclosure, heparin is used to induce differentiation, and may be used at a concentration of 0.05 to 0.5%, preferably 0.1 to 0.3%. Excessive use of heparin may rather inhibit cell growth and cause cell aging.

The number of cells inoculated in Step (c) above may be 400 to 700, 450 to 650, and 450 to 550.

Step (c) may be performed for 15 to 30 days, 17 to 30 days, and 20 to 30 days.

The respiratory organoid may include ciliated epithelial cells on the exterior of the organoid (outer layer), goblet cells, which are secretory cells, underneath thereof (middle layer), and basal stem cells on the interior of the organoid (inner layer).

The ciliated epithelial cells may exhibit motility.

Additionally, the present disclosure provides respiratory organoids prepared by the method for preparing respiratory organoids above.

Additionally, the present disclosure provides a method for evaluating the toxicity of a harmful substance, including:
(a) treating the respiratory organoid according to the present disclosure with a harmful substance;
(b) measuring the viability of cells in the respiratory organoid of the treatment group treated with the harmful substance in Step (a) and cells in the respiratory organoid of the control group that are not treated with the harmful substance; and
(c) determining the toxicity of the harmful substance by comparing the viability of cells measured in Step (b).

The harmful substance may be a toxic substance, such as fine dust, dust, smoke, air pollution, chemicals, and may be a pathogen, such as respiratory viruses or infectious bacteria.

The chemicals refer to substances that can irritate and damage the respiratory system when exposed, such as chlorine, sulfur dioxide, hydrogen sulfide, cyanide, methane, carbon monoxide, nitrogen dioxide, and/or ammonia, but are not limited thereto. When a living organism is exposed to chemicals, it can cause a burning sensation in the respiratory system and cause hemoptysis and coughing, and nausea and shortness of breath are common symptoms. In the long term, respiratory function can be impaired, and chronic coughing and shortness of breath may result.

The pathogen is a microorganism or substance that may be the cause of a disease, and may include, but is not limited to, viruses, viroids, mycoplasmas, bacteria, fungi, algae, parasitic plants, nematodes, and/or mites.

Since existing lung or respiratory organoids were prepared in a form in which epithelial cells are located in the interior of the organoids, when evaluating tissue damage caused by harmful substances, harmful substances had to be injected inside the organoids, which could result in experimental variation such as damage to the organoids during the injection process, thereby reducing accuracy. In the case of the organoids of the present disclosure, since the epithelial cells are exposed to the exterior, it is possible to evaluate the toxicity of harmful substances simply by exposing the organoids to the harmful substances.

When the survival rate of cells constituting the organoids in the treatment group exposed to the harmful substance is significantly lower, and there is a statistically significant difference between the survival rate of cells constituting the organoids in the control group, which are not exposed to the harmful substance, and the survival rate of cells constituting the organoids in the treatment group exposed to the harmful substance, it may be judged that the harmful substance is toxic. Additionally, it may be judged that the greater the difference in survival rate, the higher the degree of toxicity, and in particular, the difference may be 1.2 times or more, 1.5 times or more, 1.7 times or more, 2 times or more, or 2.3 times or more.

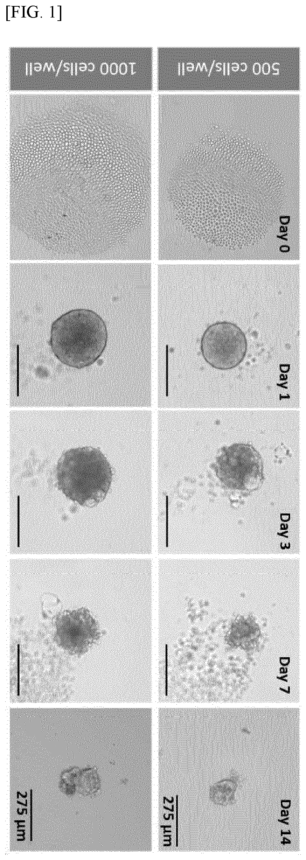

In a specific embodiment of the present disclosure, organoids were prepared by proliferating bronchial epithelial cells and inoculating 500 or 1,000 cells per well to be differentiated, and subjected to observation, and it was confirmed that the size of the prepared apical-out respiratory organoids became smaller as the differentiation period increased, and from day 7, the size was maintained at a constant level of about 100 µm. Additionally, it was confirmed that the organoids that were prepared by inoculating 500 cells firmly maintained a spherical shape (FIG. 1).

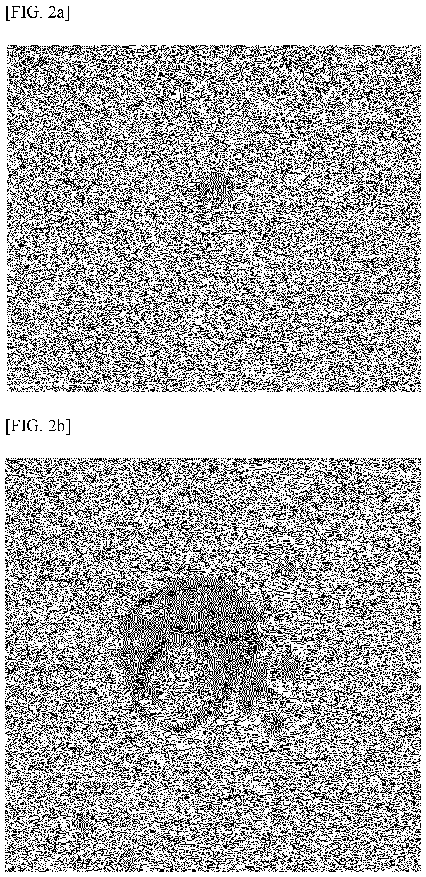

In addition, it was confirmed that ciliated cells were formed and maintained from day 14 of differentiation (FIG. 1), and as a result of observing respiratory organoids on day 21 of differentiation, it was found that ciliated structures were formed on the exterior of the organoids and that ciliated epithelial cells were functioning normally, as there was ciliary motility (beating) (FIGS. 2a and 2b).

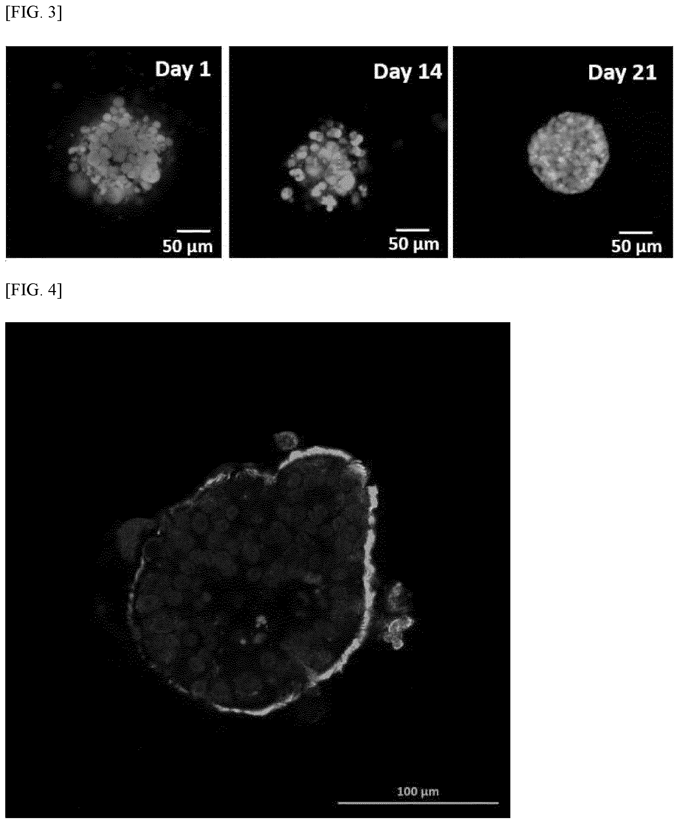

When the organoids are prepared too large, the internal cells may have difficulty communicating with the outside, and necrosis of the internal cells may occur. In order to confirm this, a live/dead staining assay was performed, and through the assay, it was observed that some cells of the respiratory organoids of the present disclosure died inside the organoids on day 21, but no necrotic core was observed (FIG. 3).

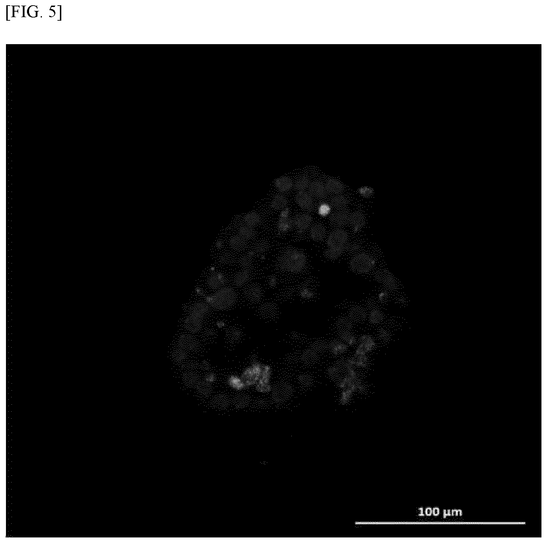

Immunofluorescence staining was performed to confirm the type of cells that make up the organoids of the present disclosure, and it was confirmed that ciliated epithelial cells were located in the outer layer of the organoids, and goblet cells were present underneath thereof (FIG. 4), and basal stem cells were located on the interior of the organoids (FIG. 5), which implies that the organoids of the present disclosure are similar to the structure of actual respiratory tissue.

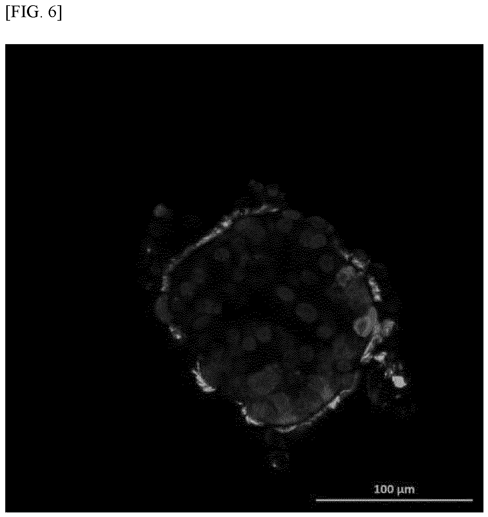

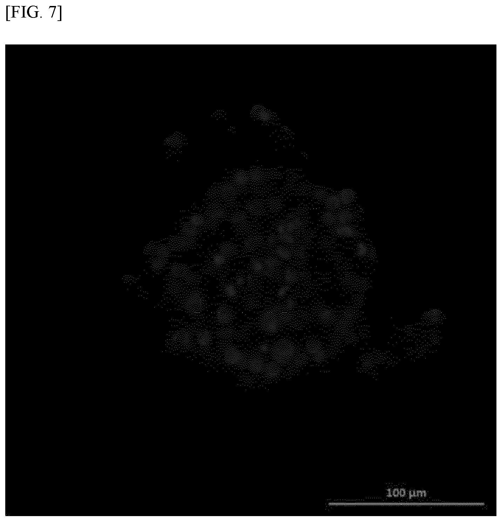

In order to confirm the importance of the composition of the additives for the preparation of organoids of the present disclosure, organoids were prepared by changing the composition of some additives, and immunofluorescence staining was performed to confirm the composition of cells. As a result, it was found that the organoids with the changed composition of the additives had a similar constitution in which the ciliary structures were located in the outer layer of the organoids and the goblet cells were located on the inner side thereof, but did not contain basal stem cells (FIGS. 6 and 7). That is, it can be seen that the cell composition of the organoids can be changed when the constitution of the composition is altered.

Based on the results, it can be seen that respiratory organoids having a structure similar to actual respiratory tissue can be prepared using the preparation method of the present disclosure, and it is expected that the respiratory organoids will better mimic actual organs, and thus respiratory injury models, *etc.* will be more accurately confirmed.

Hereinafter, the present disclosure will be described in more detail by way of specific Examples. However, these Examples are merely preferred Examples given for illustrative purposes, and thus, the scope of the present disclosure is not intended to be limited to or by these Examples.

### <Example 1> Preparation of Respiratory Organoids

After coating the cell culture plate with laminin (Laminin-511, iMatrix-511) at a concentration of 0.25 µg/cm², normal human bronchial epithelial cells (NHBE cells) were seeded and cultured for 3 days in an expansion medium to allow for expansion. The composition of the expansion medium is shown in Table 1.

When NHBE cells showed about 80% cell confluency, they were dissociated using trypsin (Trypsin-EDTA), centrifuged, and the supernatant was removed to obtain a cell pellet. The differentiation medium was then added to the cell pellet to resuspend the cells. The composition of the differentiation medium is shown in Table 1.

The number of resuspended cells was measured to be 5×10³ cells/ml, and seeded into a 96-well ultra-low attachment plate at 100 µl per well, and the plate containing the cells was centrifuged at 1000 rpm for 2 minutes to gather the cells to the center of the well. After confirming that the cells were gathered to the center under a microscope, differentiation was performed for 21 days while changing the differentiation medium every other day under conditions of 37°C and 5% CO₂. The differentiation medium replacement was performed by removing 50 µl of the existing differentiation medium per well using a pipette and adding 50 µl of a new differentiation medium per well.

**[Table 1]**

| Name | Composition |
|---|---|
| Expansion medium | 1 uM A8301 (TGF-beta/SMAD signaling inhibitor) |
| | 5 uM Y-27632 (Rock signaling inhibitor) |
| | 0.5 uM CHIR99021 (WNT signaling activator/GSK3beta signaling inhibitor) |
| | 0.5 uM SB202190 (MAPK signaling inhibitor) |
| | Bronchial epithelial growth medium (BEGM) |
| Differentiation medium | 0.96 ug/ml hydrocortisone |
| | 0.2% heparin |
| | PneumaCult-ALI medium |

### <Example 2> Observation of Morphology of Respiratory Organoids

In order to confirm the characteristics of the respiratory organoids prepared by the method according to Example 1, they were observed under a microscope.

First, in order to confirm the cell density suitable for organoid formation and maintenance, 500 or 1,000 cells were seeded per well and cultured. As a result, both groups (500/well or 1,000/well) were observed to have cell debris attached to the bottom of the well and organoids floating in the medium. When observing the morphology of the organoids of both groups, it was confirmed that the group seeded with 500 cells firmly maintained a spherical shape more than the group seeded with 1,000 cells, and therefore, 500 cells were seeded per well in the subsequent experiments (FIG. 1).

Additionally, the size of the organoids decreased over time compared to day 1 of differentiation, and on day 7 of differentiation, the size was formed to be approximately 100 µm, and the size was maintained thereafter (FIG. 1).

In the case of organoids formed by conventional methods, in particular, organoids prepared using Matrigel, the size was not uniform, and there were cases where the size was too large and nutrients were not supplied well to the cells inside the organoids. However, it can be seen that the organoids of the present disclosure were prepared while maintaining a constant size.

In addition, it was confirmed that cilia were formed on the outer edge of both groups of organoids, and it was observed that the shape was maintained after cilia were formed on day 14 of differentiation (FIG. 2). Further, when observed under a microscope, the cilia structures were found to have motility (beating).

That is, the organoids of the present disclosure were observed to have a consistent shape and consistent size of a sphere, and it was observed that not only were ciliated epithelial cells located on the exterior of the organoids, but also the cilia structures exhibited normal function.

### <Example 3> Analysis of Survival Rate of Respiratory Organoid Constituent Cells

If the interior of the organoid does not communicate with the external environment, the internal cells may die due to lack of nutrition, and organoids of this type cannot mimic the function of normal organs. In order to confirm whether the cells that make up the respiratory organoids prepared through Example 1 die, a live/dead staining assay was performed.

Specifically, the organoids were transferred to a 1.5 ml tube and left for 1 hour to allow the organoids to naturally settle to the bottom. The settled organoids were washed with Dulbecco's Phosphate-Buffered Saline (DPBS), and then Calcein-AM and ethidium-homodimer-1 were added thereto. After staining while leaving the organoids at room temperature for 1 hour, they were observed using a fluorescence microscope. Through the staining, live cells are stained green (calcein-AM) and dead cells are shown in red (ethidium-homodimer-1).

As a result, on days 1 and 14 of differentiation, most cells were observed to be alive, and no dead cells were observed. In the organoids on day 21 of differentiation, some cells in the center were observed to be dead, but most cells were observed to be alive (FIG. 3).

Additionally, when the size of the organoids is not uniform or the organoids are formed large, a necrotic core, in which the cells in the center are continuously killed and only the cells on the exterior (outer layer) survive, may occur, but in the respiratory organoids prepared by the method of Example 1, no necrotic core was observed (FIG. 3).

Accordingly, it can be predicted that the organoids of the present disclosure have stability in which cells are not killed during the differentiation process, and are maintained at an appropriate size, in which no necrotic core is observed, and that interactions between internal cells of the organoids and the external environment occur appropriately.

### <Example 4> Confirmation of Apical-out Respiratory Organoids

To confirm whether respiratory organoids were prepared with lung epithelial cells located externally, similar to the structure of the actual respiratory tract, immunofluorescence staining was performed.

Specifically, organoids were fixed with 4% paraformaldehyde for 10 minutes at room temperature, washed with DPBS, and treated with 0.5% Triton X-100 for 10 minutes to increase permeability. The organoids were treated with 3% BSA solution for 30 minutes to block nonspecific binding, and primary antibodies were diluted in 3% BSA, incubated overnight at 4°C, and then washed three times. Secondary antibodies were diluted in DPBS, left at room temperature for 1 hour, and then washed three times. As the primary antibodies, antibodies against acetylated-tubulin (Sigma, #T7451), a marker of ciliated epithelial cells, Muc5ac (Abcam, #198294), a marker of secretory cells, or KRT5 (Thermofisher, #MA5-17057), a marker of basal stem cells, were used. As the secondary antibodies, Alexafluor 488 (Thermofisher, #A11001) and Alexafluor 594 (Thermofisher, #A11012) were used. For cell nuclei, DAPI (4',6-Diamidino-2-phenylindole dihydrochloride) was diluted to 5 µg/ml in tertiary distilled water, and treated to the organoids that had completed secondary antibody staining for 3 minutes, washed, and subjected to staining.

As a result, ciliated epithelial cells (green) were observed on the outer surface of the respiratory organoids prepared in Example 1, and goblet cells (red), which are secretory cells, were observed on the inner side of the ciliated epithelial cells (FIG. 4).

Additionally, basal stem cells (green) were observed on the interior of the organoids (FIG. 5).

With reference to the structure of the actual respiratory organ, ciliated epithelial cells are located at the most apical part exposed to the air, mucus-secreting cells such as goblet cells are located underneath the epithelial cells, and basal stem cells exist at the innermost part as reserve cells for epithelial cell replacement. In this regard, it was confirmed that the organoid structure of the present disclosure has a structure closer to the structure of the actual respiratory tissue.

### <Example 5> Test of Medium Composition

In order to confirm the characteristics of the organoids according to the medium additives of the present disclosure, respiratory organoids were prepared in the same manner as the method of Example 1 with the medium composition as in Table 2, in which CHIR99021 was excluded and the concentration of Y-27632 was altered, and immunofluorescence staining was performed as in Example 4 to observe the constituent cells of the organoids.

**[Table 2]**

| Name | Composition |
|---|---|
| Modified expansion medium | 1 uM A8301 (TGF-beta/SMAD signaling inhibitor) |
| | 1 uM Y-27632 (Rock signaling inhibitor) |
| | 0.5 uM SB202190 (MAPK signaling inhibitor) |
| | Bronchial epithelial growth medium (BEGM) |
| Differentiation medium | 0.96 ug/ml hydrocortisone |
| | 0.2% heparin |
| | PneumaCult-ALI medium |

As a result, it was similarly observed that ciliated epithelial cells were distributed on the exterior of the organoids and secretory cells (goblet cells) were distributed on the interior (FIG. 6), but basal stem cells were not maintained on the interior of the organoids (FIG. 7).

Therefore, it was confirmed that the composition of the expansion medium shown in Table 1 is suitable for preparing apical-out respiratory organoids in which basal stem cells are located on the interior, ciliated epithelial cells are located on the exterior, and secretory cells are located underneath the ciliated epithelial cells.

Based on the Examples, apical-out respiratory organoids can be prepared when the medium composition of the present disclosure was employed and the cells were proliferated and differentiated in an environment coated with laminin rather than Matrigel. Additionally, the respiratory organoids can be prepared as organoids that mimic the actual respiratory tissues, in which ciliated structures are present on the exterior of the organoids, secretory cells are located on the inner side thereof, and basal stem cells are present on the interior.

## Claims

1. A method for preparing a respiratory organoid, in which ciliated epithelial cells are present on the exterior, comprising:
(a) culturing respiratory cells using an expansion medium on a plate coated with an extracellular matrix material;
(b) obtaining the cultured cells of Step (a) and removing the expansion medium by centrifugation, thereby obtaining a cell pellet; and
(c) resuspending the cell pellet in a differentiation medium, and then inoculating and culturing a certain number of cells on a plate to be differentiated.

2. The method of claim 1, wherein the respiratory organoid is a lung organoid.

3. The method of claim 1, wherein the respiratory cell is a normal human bronchial basal epithelial cell (NHBE cell).

4. The method of claim 1, wherein the extracellular matrix material of Step (a) is laminin, collagen I, or collagen IV.

5. The method of claim 4, wherein the extracellular matrix material of Step (a) is laminin.

6. The method of claim 1, wherein the expansion medium used in Step (a) comprises A8301, Y-27632, CHIR99021, and SB202190.

7. The method of claim 6, wherein the expansion medium comprises a bronchial epithelial expansion medium (BEGM), 2 µM of A8301, 2 to 10 µM of Y-27632, 0.5 to 3 µM of CHIR99021, and 0.1 to 2 µM of SB202190.

8. The method of claim 1, wherein the number of cells inoculated in Step (c) is 400 to 700.

9. The method of claim 1, wherein Step (c) is performed for 15 to 30 days.

10. The method of claim 1, wherein the respiratory organoid comprises basal stem cells on the interior of the organoid.

11. A respiratory organoid prepared by the method of claim 1.

12. The respiratory organoid of claim 11, wherein the respiratory organoid has a constitution in that ciliated cells are located on the exterior of the organoid, goblet cells, which are secretory cells, are located inner side thereof, and basal stem cells are located on the interior of the organoid.

13. A method for evaluating the toxicity of a harmful substance, comprising:
(a) treating a respiratory organoid prepared by the method of claim 1 with a harmful substance;
(b) measuring the viability of cells in the respiratory organoid of the treatment group treated with the harmful substance in Step (a) and cells in the respiratory organoid of the control group which are not treated with the harmful substance; and
(c) determining the toxicity of the harmful substance by comparing the viability of cells measured in Step (b).
